# EUROPEAN PATENT APPLICATION

(11) **EP 3 622 955 A1**
(43) Date of publication of application: **18.03.2020**
(21) Application number: 18194909.0
(22) Date of filing: 17.09.2018
(51) Int. Cl.: A61K 31/4453, A61K 9/00, A61P 25/36, A61P 39/02, A61P 43/00

(54) **BIPERIDEN HCL FOR TREATING BRAIN DISORDERS AS A CONDEQUENCE OF DRUG DETOXIFICATION**

(71) Applicant: Cheng, En-Che, 830 Kaohsiung City (TW)
(72) Inventor: Cheng, En-Che, 830 Kaohsiung City (TW)
(74) Representative: Cabinet Chaillot

(57) **Abstract**

A purpose of Biperiden Hcl(10) is mainly to be provided to the patient to sooth brain disorder of the patient after the patient is treated with drug detoxification such that consciousness of the patient can be recovered to rapidly become normally physiological state.

## Description

### BACKGROUND OF THE INVENTION

### Field of the Invention

The present invention relates to a purpose of Biperiden Hcl, and more particularly to an issue that provides Biperiden Hcl medicine to patients after curing patients with drug detoxification so that brain disorders occurring on patients can be soothed from drug detoxification to recover consciousness of patients, thereby rapidly becoming normally physiological state.

### Description of the Related Art

U.S. utility patent No. 10004781 invented and filed by applicant(s) disclose a feature of mainly blocking poisoned path of drug (such as heroin or morphine) and that is a treatment prescription. However, a drug abuser as a patient frequently has brain disorder after experiencing drug detoxification, resulting in situation that is unable to rapidly recover consciousness. Thus, the inventor(s) developed the medicine based upon foregoing situation.

### SUMMARY OF THE INVENTION

In view of the aforementioned drawbacks of the prior art, the inventor of the present invention conducted researches and experiments, and finally developed a medicine for soothing brain disorders of patients from drug detoxification so that patients' consciousness can be recovered to rapidly become normal physiological state.

Therefore, it is a primary objective of the present invention to provide a Biperiden Hcl medicine to patients in order to soothe brain disorders of patients from drug detoxification so that patients' consciousness can be recovered to rapidly become normal physiological state.

### BRIEF DESCRIPTION OF THE DRAWINGS

Fig. 1 is an application flow chart according to the embodiment of the present invention; and
Fig. 2 is an application flow chart comprising Mecobalamin medicine according to the embodiment of the present invention.

### DETAILED DESCRIPTION OF THE PREFERRED EMBODIMENTS

The technical characteristics, contents, advantages and effects of the present invention will be apparent with the detailed description of a preferred embodiment accompanied with related drawings as follows.

A Biperiden Hcl 10 medicine of the disclosure is a cure of Parkinson's disease and can reduce effect of acetylcholine of Extrapyramidal Symptoms (EPS) on it to further decrease excitement of central nervous. Thus, it has the most effective with regard to akinesia and ankylosing spondylitis and also has effect on drooling and seborrhea to generate temporarily euphoria and improve emotion. Moreover, the inventor(s) found that Biperiden Hcl 10 can soothe brain disorders of patients after patients are treated with block-out of poisoning path by providing the Biperiden Hcl 10. Such medicine has excellent effect of soothing brain disorders of patients and recovering consciousness and normal physiological state.

The invention refers to Biperiden Hcl 10 for use in soothing brain disorder of a patient from drug detoxification, the Biperiden Hcl 10 being a medicine provided to the patient after experiencing with a poisoning path treatment 20.

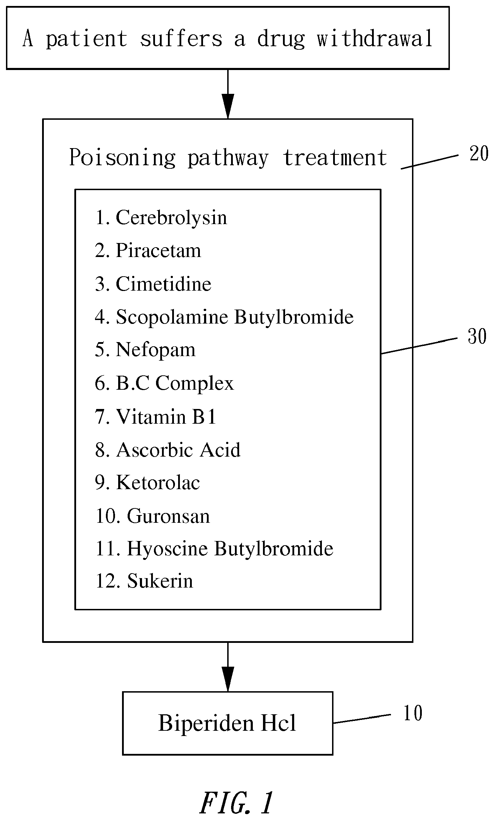

With reference to Fig. 1, a Biperiden Hcl 10 medicine is applied after patients are treated with block-out of poisoning path, wherein poisoning path treatment 20 can adapt intravenous drip prescription 30 disclosed in U.S. utility patent application No. 10004781 owned by the inventor(s) and purpose treatment thereof, providing the intravenous drip prescription 30 to patients. The intravenous drip prescription 30 comprises a plurality of ingredients consisting of Cerebrolysin, Piracetam, Cimetidine, Scopolamine Butylbromide, Nefopam, B. C Complex, Vitamin B1, Ascorbic acid, Ketorolac, Guronsan, Hyoscien Butylbromide, and Sukerin.

After a patient is dealt with poisoning path treatment 20, a dose (10mg/ml) of Biperiden Hcl 10 is provided to the patient to sooth brain disorder of the patient from the poisoning path treatment 20 of the patient, capable of rapidly recovering consciousness and normal physiological state.

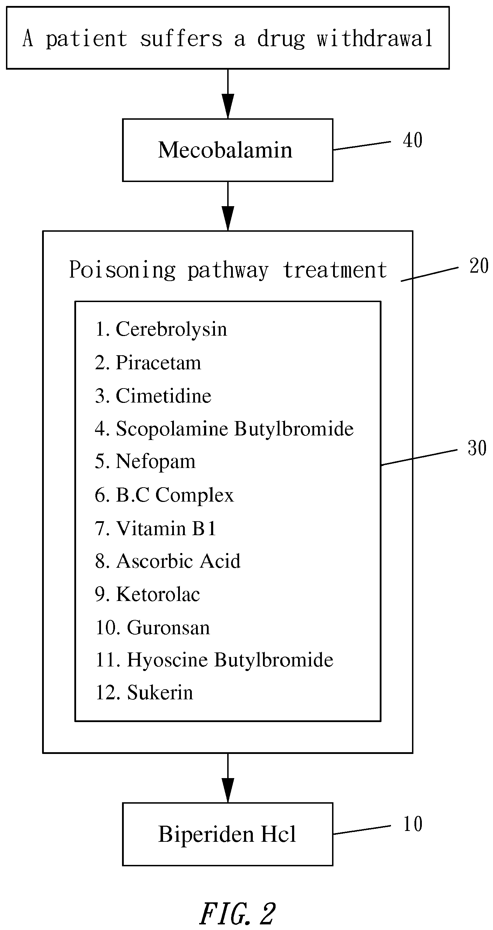

Next, with reference to Fig. 2, when the drug abuser is going through withdrawal, Mecobalamin 40 medicine (a dose of 250-500mg/ml) is provided to the patient. Drug abuser as the patient can be firstly soothed by the Mecobalamin 40 to relieve pain of the patient. Afterward the poisoning path treatment 20 is carried out.

Accordingly, when the drug abuser as the patient is going through withdrawal, the Mecobalamin 40 medicine is provided to the patient before carrying out poisoning path treatment 20. The drug addiction symptom of the patient is firstly soothed by the Mecobalamin 40 to relieve pain of the patient. After the patient is treated with poisoning path treatment 20, a dose of Biperiden Hcl 10 is provided to the patient to relieve brain disorder of the treated patient such that the patient can be rapidly recovered with consciousness and normal physiological state to produce extremely excellent effect on treatment.

While the present invention has been described by means of specific embodiments, numerous modifications and variations could be made thereto by those skilled in the art without departing from the scope and spirit of the invention set forth in the claims.

## Claims

1. Biperiden Hcl(10) for use in soothing brain disorder of a patient from drug detoxification, the Biperiden Hcl(10) being a medicine provided to the patient after experiencing with a poisoning path treatment(20).

2. Biperiden Hcl(10) for use according to claim 1, wherein the poisoning path treatment(20) is prepared with an intravenous drip prescription(30), the intravenous drip prescription(30) comprising a plurality of ingredients consisting of Cerebrolysin, Piracetam, Cimetidine, Scopolamine Butylbromide, Nefopam, B.C Complex, Vitamin B1, Ascorbic Acid, Ketorolac, Guronsan, Hyoscine Butylbromide, and Sukerin.

3. Biperiden Hcl(10) for use according to claim 1, further comprising a Mecobalamin(40) medicine, wherein the Mecobalamin(40) is provided to the patient before experiencing the poisoning path treatment(20).

4. Biperiden Hcl(10) for use according to claim 2, further comprising a Mecobalamin(40) medicine, wherein the Mecobalamin(40) is provided to the patient before experiencing the poisoning path treatment(20).

5. Biperiden Hcl(10) for use according to claim 1, wherein a dose of the Biperiden Hcl(10) provided to the patient is 10mg/ml.

6. Biperiden Hcl(10) for use according to claim 3, wherein a dose of the Mecobalamin(40) is 250-500mg/ml.

7. Biperiden Hcl(10) for use according to claim 4, wherein a dose of the Mecobalamin(40) is 250-500mg/ml.
